(19) Europäisches Patentamt
European Patent Office
Office européen des brevets

(11) Veröffentlichungsnummer: **0 531 689 B1**

(12) # EUROPÄISCHE PATENTSCHRIFT

(45) Veröffentlichungstag der Patentschrift: **22.03.95**

(21) Anmeldenummer: **92112894.8**

(22) Anmeldetag: **01.08.92**

(51) Int. Cl.[6]: **C07C 273/18**, C07D 295/20, C07C 275/28, C07C 275/30, C07C 275/34, C07C 275/40, C07D 295/215

(54) **Verfahren zur Herstellung reiner N,N'unsymmetrisch substituierter Phenylharnstoffe.**

(30) Priorität: **16.08.91 AT 1616/91**

(43) Veröffentlichungstag der Anmeldung:
**17.03.93 Patentblatt 93/11**

(45) Bekanntmachung des Hinweises auf die Patenterteilung:
**22.03.95 Patentblatt 95/12**

(84) Benannte Vertragsstaaten:
**AT BE CH DE DK ES FR GB IE IT LI NL SE**

(56) Entgegenhaltungen:
**GB-A- 816 855**
**GB-A- 2 170 808**
**US-A- 2 673 877**

**CHEMICAL ABSTRACTS, vol. 110, no. 21, 22. Mai 1989, Columbus, Ohio, US; abstract no. 192451g, K. MOHAN ET AL. 'Process for the preparation of 3-(4-isopropylphenyl)-1,1-dimethyl urea, i.e. the herbicide isoproturon.' Seite 710 ;Spalte 2 ;**

**CHEMICAL ABSTRACTS, vol. 59, no. 1, 8. Juli 1963, Columbus, Ohio, US; abstract no. 485e, M. YASUHIKO 'Reactions of amines with urea and its derivatives. IV. Reactions of mono-substituted ureas with amines.' Spalte 485 ;**

(73) Patentinhaber: **Chemie Linz GmbH**
**St.Peter-Strasse 25**
**A-4021 Linz (AT)**

(72) Erfinder: **Hackl, Kurt Alfred, Dipl.-Ing. Dr.**
**Anzengruberstrasse 3**
**A-4020 Linz (AT)**
Erfinder: **Falk, Heinz, Dr.**
**Leonfeldnerstrasse 151/13**
**A-4040 Linz (AT)**

(74) Vertreter: **Kunz, Ekkehard, Dr.**
**Chemie Linz GmbH**
**Abteilung Patente**
**St. Peter-Str. 25**
**A-4021 Linz (AT)**

**EP 0 531 689 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung reiner N,N'-unsymmetrisch substituierter Phenylharnstoffe aus Phenylharnstoffen und Aminen.

N,N'-unsymmetrisch substituierte Phenylharnstoffe sind biologisch aktiv und werden beispielsweise als Herbizide eingesetzt. Ein Verfahren zur Herstellung N,N'-unsymmetrisch substituierter Phenylharnstoffe aus Alkylharnstoff und gegebenenfalls substituiertem Amin ist beispielsweise aus DE-PS 1 064 051 bekannt. Dort ist beschrieben, daß Harnstoff mit einer äquivalenten Menge eines Mono- oder Dialkylamins in wäßriger Lösung umgesetzt und der Alkylharnstoff mit einer äquimolaren Menge eines Anilins erwärmt wird, bis die Ammoniakentwicklung aufgehört hat.
Gemäß DE-OS-36 02 657 können N,N'-unsymmetrisch substituierte Phenylharnstoffe durch gleichzeitige Umsetzung von Harnstoff mit einem Anilin und einem entsprechenden sekundären Amin in einem hydroxylgruppenfreien Lösungsmittel hergestellt werden.
In SU-178 367 wird die Herstellung von N-p-Chlorphenyl-N'-N'-dimethylharnstoff durch Reaktion von Harnstoff in Trichlorbenzol mit einem Überschuß an p-Chloranilin und anschließendem Einleiten eines großen Überschusses Dimethylamin in das Reaktionsgemisch bei Temperaturen von 175 bis 202 °C beschrieben.

In US-A-2,673,877 ist die Umsetzung eines 3-(Chlorphenyl)harnstoffs mit Dimethylamin in einem inerten Verdünnungsmittel bei Temperaturen von 135 bis 225 °C geoffenbart.
GB-A-816,855 betrifft unter anderem ein Verfahren zur Herstellung von N-(4-Bromphenyl)-N'-(4-Nitrophenyl)harnstoff durch Umsetzung von N-(4-Bromphenyl)harnstoff mit p-Nitroanilin in einem inerten Lösungsmittel bei Temperaturen von etwa 180 °C. Aus Chemical Abstracts, Volume 110 : 192451g geht ein Verfahren zur Herstellung von N-(4-Isopropylphenyl)-N', N'-dimethylharnstoff durch Umsetzung von N-(4-Isopropyl)harnstoff mit Dimethylamin in Cumol bei Temperaturen von 130 - 140 °C hervor.

Die Produkte, die nach den oben angegebenen Verfahren entstehen, sind aber immer durch Nebenprodukte, die schwer zu entfernen sind, verunreinigt. Dies ist auch nicht verwunderlich, denn gemäß Davis et al., J.Am.Chem.Soc. 44, 2595, 1922 wird beim Erwärmen von Harnstoff mit einem Amin Isocyansäure und beim Erwärmen eines monosubstituierten Harnstoffes mit einem Amin das entsprechende Isocyanat als Zwischenprodukt gebildet, bevor die Umsetzung zum N,N'-unsymmetrisch substituierten Phenylharnstoff erfolgen kann. Da Isocyansäure und Isocyanate sehr reaktionsfähig sind und unspezifisch reagieren, muß es daher bereits von Anfang der Umsetzung an zur Bildung von Nebenprodukten, beispielsweise Biuretderivaten, kommen. Solche Nebenprodukte, die den gewünschten Phenylharnstoff verunreinigen, sind im allgemeinen nur sehr schwer, wenn überhaupt, abzutrennen.

Es wurde nun unerwarteterweise gefunden, daß die Reaktion von Phenylharnstoffen mit einem Amin in einem inerten Lösungsmittel bei Temperaturen von 100 bis 200 °C nicht über das Zwischenprodukt Isocyanat, das Anlaß zur Bildung verschiedenster Nebenprodukte gibt, sondern direkt durch nucleophile Substitution verläuft. Solange die Reaktionsgeschwindigkeit, die mit der Konzentration der Ausgangsstoffe verknüpft ist, nicht absinkt, solange also die Konzentration der Ausgangsstoffe noch genügend groß ist, treten dabei keinerlei Nebenprodukte auf. Bis zu dem Zeitpunkt, an dem die Reaktionsmischung zu sehr an Ausgangsstoffen verarmt, liegen in der Reaktionsmischung stets nur reine Ausgangsstoffe, reines Endprodukt und das verwendete Lösungsmittel vor.

Wird die Umsetzung von Phenylharnstoffen mit einem Amin daher vor der Verarmung der Reaktion an Ausgangsstoffen abgebrochen, erhält man ein Gemisch reiner Ausgangsstoffe und des reinen Endproduktes, das praktisch frei von Nebenprodukten ist. Da ein N,N'-unsymmetrisch substituierter Phenylharnstoff aufgrund seiner spezifischen chemischen Konstitution sehr leicht von den Ausgangsstoffen, das heißt vom Phenylharnstoff und vom Amin abgetrennt werden kann, wird auf diese Weise sehr reiner, N,N'-unsymmetrisch substituierter Phenylharnstoff erhalten. Die nicht umgesetzten Ausgangsverbindungen, die ebenfalls in sehr reiner Form vorliegen, werden nach Abtrennung des gebildeten N,N'-substituierten Phenylharnstoffes wieder miteinander zur Reaktion gebracht, sodaß letztendlich sehr reiner N,N'-unsymmetrisch substituierter Phenylharnstoff praktisch quantitativ hergestellt werden kann. Wesentlich ist bei dieser Methode, daß Phenylharnstoff mit einem Amin und nicht ein Alkylharnstoff mit einem Anilin umgesetzt wird. Die Reaktion von einem Alkylharnstoff mit einem Anilin verläuft nämlich im allgemeinen erst bei höheren Temperaturen als die Reaktion von Phenylharnstoff mit einem Alkylamin und es entstehen von Anfang an Nebenprodukte.

Gegenstand der Erfindung ist daher ein Verfahren zur Herstellung reiner N,N'-unsymmetrisch substituierter Phenylharnstoffe der Formel

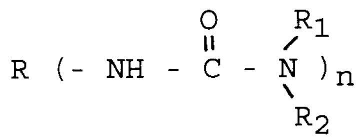

(I)

in der R eine unsubstituierte oder durch unter den Reaktionsbedingungen inerte Gruppen substituierte Phenyl- oder Phenylengruppe und $R_1$ und $R_2$ entweder gleich sind und jeweils eine Alkylgruppe, oder verschieden sind, wobei $R_1$ ein Wasserstoffatom oder eine Alkylgruppe und $R_2$ eine Alkyl- oder Phenylgruppe oder $R_1$ und $R_2$ gemeinsam mit dem Stickstoffatom einen heterocyclischen Ring, der keine ungesättigten Bindungen enthält, und n die Zahl 1 oder 2 bedeuten, durch Umsetzung eines Phenylharnstoffs der Formel

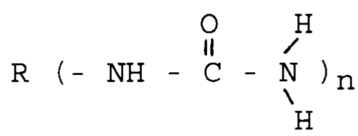

(II)

in der R und n die oben angegebene Bedeutung haben, mit einem Amin der Formel

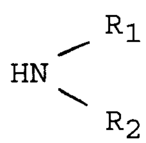

(III)

in der $R_1$ und $R_2$ die oben angegebenene Bedeutung haben, in einem unter den Reaktionsbedingungen inerten Verdünnungsmittel bei Temperaturen von 100 bis 200 °C, das dadurch gekennzeichnet ist, daß die Umsetzung, noch bevor Nebenprodukte entstehen, abgebrochen wird, worauf der N,N'-unsymmetrisch substituierte Phenylharnstoff der Formel I aus dem Reaktionsgemisch abgetrennt und die nicht umgesetzten Ausgangsverbindungen der Formeln II und III gegebenenfalls erneut in das Verfahren eingesetzt werden.

In der Formel I bedeutet R eine unsubstituierte oder durch unter den Reaktionsbedingungen inerte Gruppen substituierte Phenyl- oder Phenylengruppe. Unter den Reaktionsbedingungen inerte Gruppen sind beispielsweise Halogenatome, Alkylgruppen, Arylgruppen, Alkoxygruppen, Aryloxygruppen, Aminogruppen, die durch unter den Reaktionsbedingungen inerte Gruppen wie Alkyl- oder Arylgruppen substituiert sind, Nitrogruppen. Die Alkyl-, Aryl-, Alkoxy- oder Aryloxygruppen können ihrerseits durch inerte Gruppen, wie sie oben angegeben sind, substituiert sein. Unter Halogenatomen sind insbesonders Fluor, Chlor oder Brom, unter Alkyl- oder Alkoxygruppen geradkettige, verzweigte oder cyclische Alkyl- oder Alkoxygruppen zu verstehen.

Bevorzugt bedeutet R eine unsubstituierte oder durch Halogenatome, Alkylgruppen mit 1 bis 6 C-Atomen, die Trifluormethylgruppe, Alkoxy- oder Dialkylaminogruppen, wobei die Alkylgruppen 1 bis 6 C-Atome, besonders bevorzugt 1 bis 3 C-Atome, aufweisen, substituierte Phenyl- oder Phenylengruppe.

$R_1$ und $R_2$ sind entweder gleich und bedeuten jeweils eine Alkylgruppe, die unsubstituiert oder durch unter den Reaktionsbedingungen inerte Gruppen, wie oben angegeben, substituiert ist oder $R_1$ und $R_2$ sind verschieden und $R_1$ bedeutet ein Wasserstoffatom oder eine Alkylgruppe und $R_2$ eine Alkyl- oder Phenylgruppe, wobei die Alkygruppen und/oder die Phenylgruppen unsubstituiert oder durch unter den Reaktionsbedingungen inerte Gruppen, wie sie oben angegeben sind, substituiert sein können, oder $R_1$ und $R_2$ bedeuten gemeinsam mit dem Stickstoffatom einen heterocyclischen Ring, der keine ungesättigten Bindungen enthält, der durch weitere Heteroatome durchbrochen sein und der unsubstituiert oder durch unter den Reaktionsbedingungen inerten Gruppen, wie oben angegeben, substituiert sein kann. Beispiele für solche heterocyclische Ringe sind etwa der Pyrrolidin-, Piperidin-, Piperazin-, Morpholin-, Thiazolidin-, Thiomorpholinring. n bedeutet die Zahl 1 oder 2. Unter Alkylgruppen sind dabei geradkettige, verzweigte oder cyclische, bevorzugt geradkettige oder verzweigte Alkylgruppen zu verstehen.

Wenn $R_1$ und $R_2$ gleich sind, bedeuten $R_1$ und $R_2$ bevorzugt unsubstituierte, geradkettige Alkylgruppen mit

1 bis 18, besonders bevorzugt mit 1 bis 12 C-Atomen. Wenn $R_1$ und $R_2$ verschieden sind, bedeutet $R_1$ bevorzugt ein Wasserstoffatom und $R_2$ eine geradkettige Alkylgruppe mit 1 bis 22 C-Atomen, eine verzweigte Alkylgruppe mit 1 bis 8, besonders bevorzugt mit 1 bis 6 C-Atomen oder eine unsubstituierte oder durch Halogenatome, Alkylgruppen mit 1 bis 6 C-Atomen, Alkoxy- oder Dialkylaminogruppen, wobei die Alkylgruppen 1 bis 6 C-Atome aufweisen, substituierte Phenylgruppe. Wenn $R_1$ und $R_2$ gemeinsam mit dem Stickstoffatom einen heterocyclischen Ring, der keine ungesättigten Bindungen enthält, bedeuten, ist der Pyrrolidin-, Piperidin- oder Morpholinring besonders bevorzugt.

Zur Herstellung der N,N'-unsymmetrisch substituierten Phenylharnstoffe der Formel I, wird ein Phenylharnstoff der Formel II mit einem Amin der Formel III in einem unter den Reaktionsbedingungen inerten Verdünnungsmittel bei Temperaturen von 100 bis 200 °C reagieren gelassen.
Phenylharnstoffe der Formel II können beispielsweise aus entsprechend substituierten Anilinen durch Umsetzung mit Isocyansäure, beispielsweise nach der in EP 0 410 168 geoffenbarten Verfahrensweise, hergestellt werden. Amine der Formel III sind bekannt oder nach bekannten Methoden erhältlich.
Der Phenylharnstoff der Formel II wird in ein unter den Reaktionsbedingungen inertes Verdünnungsmittel eingebracht und mit einem Amin der Formel III versetzt. Das Amin kann dabei als solches, fest, flüssig oder gasförmig oder in einem inerten Verdünnungsmittel gelöst, zugegeben werden. Das Amin der Formel III wird bevorzugt äquivalent zum Phenylharnstoff der Formel II eingesetzt, wobei jedoch ein Überschuß des einen oder anderen Reaktionspartners von Vorteil sein kann. Wird ein Harnstoff der Formel II, in dem n die Zahl 2 bedeutet, eigesetzt, werden ebenfalls bevorzugt äquivalente Mengen eingesetzt, d. h. es wird pro Mol freie Aminogruppe ein Mol Amin der Formel III verwendet. Als Verdünnungsmittel kommen unter den Reaktionsbedingungen inerte Verdünnungsmittel mit einem Siedepunkt von über 100 °C in Frage. Beispiele für solche Verdünnungsmittel sind Kohlenwasserstoffe, halogenierte Kohlenwasserstoffe, bevorzugt aromatische Kohlenwasserstoffe, besonders bevorzugt Toluol oder Xylole.

Die Reaktionsmischung wird vorteilhafterweise unter kräftigem Rühren gegebenenfalls unter Druck auf Temperaturen von 100 bis 200 °C, bevorzugt von 110 bis 160 °C, besonders bevorzugt auf Rückflußtemperatur des verwendeten inerten Verdünnungsmittels erhitzt. Wenn die Reaktion unter Druck durchgeführt wird, ist die Durchführung in einem Autoklaven unter Autogendruck bevorzugt.

Dabei bildet sich der Harnstoff der Formel I und pro Mol gebildeter Harnstoff ein Mol Ammoniak, ohne daß vorerst irgendwelche Nebenprodukte entstehen. In der Reaktionsmischung sind von Beginn der Reaktion an nur reines Endprodukt neben reinen Ausgangsverbindungen, das verwendete Lösungsmittel und gegebenenfalls Ammoniak vorhanden. Erst ab jenem Zeitpunkt, an dem die Reaktion zu sehr an Ausgangstoffen verarmt, werden Nebenprodukte gebildet. Dieser Zeitpunkt ist von der Art des Lösungsmittels, der Reaktionstemperatur, sowie von der Anfangskonzentration, vom molaren Verhältnis und von der chemischen Natur der jeweiligen Ausgangsverbindungen abhängig und kann für jede gewünschte erfindungsgemäße Reaktion leicht durch einen Vorversuch, der analytisch, beispielsweise chromatographisch, insbesonders gaschromatographisch, verfolgt wird, bestimmt werden. Die erfindungsgemäße Umsetzung wird dann wie im Vorversuch durchgeführt und kurz vor Erreichen des im Vorversuch ermittelten Zeitpunktes, ab dem Nebenprodukte entstehen, abgebrochen.

Durch den vorzeitigen, willkürlichen Abbruch der Reaktion entstehen im Verlauf des Verfahrens praktisch keine Nebenprodukte.

Das im Verlauf der Reaktion gebildete Ammoniak wird entweder kontinuierlich als Gas abgeführt, oder falls die Reaktion unter Druck ausgeführt wurde, nach dem Ende der Reaktion entfernt. Das Ammoniak kann aufgefangen und einer üblichen Verwendung zugeführt werden.

Zum Studium des Reaktionsmechanismus wurde 4-Methoxyphenylharnstoff in siedendem Toluol erhitzt. Dabei bildete sich nicht, wie zu erwarten gewesen wäre, 4-Methoxyphenylisocyanat, sondern der 4-Methoxyphenylharnstoff blieb völlig unverändert. Beim Erhitzen von 4-Methoxyphenylharnstoff im siedenden Toluol unter Zugabe eines Äquivalentes Dioctylamin bildet sich nach einer Stunde N,N-Dioctyl-N'-4-methoxyphenylharnstoff in 60%iger Ausbeute, wobei keine Nebenprodukte, insbesonders keine Isocyanate oder daraus hervorgehende Nebenprodukte auftraten. Der Nachweis, daß die Reaktion nicht über Isocyanate verläuft, erfolgte mit Hilfe von $^1$H-NMR bzw. $^{13}$C-NMR Spektroskopie.

Zur Bestimmung der Reaktionsordnung wurden kinetische Untersuchungen bei der Reaktion von Phenylharnstoff mit Dioctylamin im siedenden Toluol durchgeführt. Der Reaktionsmischung wurden nach bestimmten Zeitabständen Proben entnommen, das Lösungsmittel abgedampft und der Rückstand in $CDCl_3$ aufgenommen und digeriert. Da Phenylharnstoff in deuteriertem Chloroform praktisch nicht löslich ist, Dioctylamin und N-Phenyl-N',N'-dioctylharnstoff aber sehr wohl, konnte im $CDCl_3$-Extrakt mittels $^1$H-NMR-Spektroskopie direkt das jeweilige molare Verhältnis des Dioctylamis und des N-Phenyl-N',N'-dioctylharnstoffes durch Integration der Signale der $N\text{-}CH_2$-Protonen (Dioctylamin 2,57 ppm, N-Phenyl-N',N'-dioctylharnstoff 3,28 ppm) und somit das Maß des Umsatzes bestimmt werden.

Bei Auswertung der kinetischen Untersuchung gemäß A.A.Frost und R.G. Pearson, "Kinetik und Mechanismus homogener Reaktionen", Verlag Chemie, Weinheim, 1984, zeigte sich, daß die Reaktionsgeschwindigkeit sowohl von der Konzentration des Dioctylamins als auch von der des Phenyharnstoffes abhängig ist und daß sich die Meßergebnisse gut mit einem Reaktionsmechanismus zweiter Ordnung korrelieren lassen. Auch alle anderen Beobachtungen wiesen auf eine direkte nucleophile Substitution (bimolekulare Reaktion) hin, wobei gefunden wurde, daß folgender Zusammenhang besteht:

$$\frac{d\,(\text{N'-Phenyl-N,N-dioctylharnstoff})}{dt} = k_2 \cdot (\text{Phenylharnstoff}) \cdot (\text{Dioctylamin})$$

wobei $k_2$ in diesem Fall etwa 0,3 l $Mol^{-1}.sec^{-1}$ beträgt. Die Formel gilt für Konzentrationen von etwa 0,1 bis 0,2 Mol pro Liter. Die Reaktion verläuft also als Reaktion 2. Ordnung.

Das Abbrechen der Reaktion erfolgt durch Beendigen des Erhitzens und gegebenenfalls durch Kühlen des Reaktionsgemisches. Nach Abbrechen der Reaktion wird, gegebenenfalls nach dem Austreiben von noch anwesendem Ammoniak, der gebildete N,N'-unsymmetrisch substituierte Harnstoff von den nicht umgesetzten Ausgangsverbindungen der Formeln II und III abgetrennt.

In vielen Fällen genügt es dazu, die Reaktionsmischung abzukühlen. Dabei fällt nämlich häufig der Phenylharnstoff der Formel II aus, während der N,N'-unsymmetrisch substituierte Harnstoff und das Amin der Formel III in Lösung bleiben. Nach dem Abfiltrieren des Harnstoffes der Formel II kann das Amin der Formel III anschließend extraktiv oder destillativ entfernt werden, wobei der N,N'-unsymmetrisch substituierte Harnstoff der Formel I in reiner Form anfällt.

Die Reaktionsmischung kann aber auch extraktiv aufgetrennt werden. N-Phenyl-N'-alkyl- und N-Phenyl-N'-dialkylharnstoffe sind ab einer Kettenlänge der Alkylgruppen von 3-C-Atomen beispielsweise in Chloroform löslich, während N-Phenylharnstoffe schwerlöslich sind. N-Phenyl-N'-Phenylharnstoff läßt sich in der Regel durch Extraktion mit Wasser vom Phenylharnstoff abtrennen. Ferner kann die Reaktionsmischung auch mit Hilfe chromatographischer Methoden, beispielsweise durch Säulenchromatographie oder gegebenenfalls durch kristallographische Methoden, etwa fraktioniertes Kristallisieren, aufgetrennt werden.

Die Reaktion kann diskontinuierlich oder kontinuierlich durchgeführt werden.

Da im Verlauf der erfindungsgemäßen Reaktionsdurchführung keinerlei Nebenprodukte gebildet werden, fällt der N,N'-unsymmetrisch substituierte Harnstoff in sehr reiner Form an. Die Ausgangsprodukte der Formeln II und III, die im Reaktionsgemisch in sehr reiner Form verbleiben, werden nach der Abtrennung des Produktes aus dem Reaktionsgemisch wieder in die erfindungsgemäße Reaktion eingesetzt.

Auf die beschriebene Art der Reaktionsführung können N,N'unsymmetrisch substituierte Phenylharnstoffe ohne Nebenprodukte und daher sehr rein und praktisch quantitativ hergestellt werden. Das Verfahren stellt somit eine Bereicherung der Technik dar.

## Beispiel 1

1,36 g Phenylharnstoff (10 mMol) und 2,41 g Dioctylamin (10 mMol) wurden unter kräftigem Rühren in 50 ml Xylol auf Rückfluß erhitzt. Nach 30 Minuten war ein Umsatz von 70 % und nach 60 Minuten von 83 % erreicht, ohne daß Nebenprodukte gebildet worden waren. Das im Verlauf der Reaktion gebildete Ammoniak wurde gasförmig abgeführt.

Nach 60 Minuten wurde die Reaktion abbgebrochen und das Lösungsmittel abgedampft. Der Rückstand wurde in Chloroform aufgenommen und der unlösliche Phenylharnstoff abfiltriert. Aus dem Filtrat wurde das Chloroform abgedampft und das nicht umgesetzte Dioctylamin durch Hochvakuumdestillation entfernt.

Dabei wurden 3,00 g **N-Phenyl-N',N'-dioctylharnstoff**, das sind 83 % der Theorie, mit praktisch 100%iger Reinheit erhalten.

$^1$H-NMR (200 MHz; $CDCl_3$; TMS): 7,378 (m; 2H; Phenyl-2 und 6; $J_{ortho23}$ = 8,4 Hz; $J_{meta}$ = 1,3 Hz); 7,264 (m; 2H; Phenyl-3 und 5; $J_{ortho23}$ = 8,4 Hz; $J_{ortho34}$ = 7,2 Hz); 7,002 (m; 1H, Phenyl-4; $J_{meta}$ = 1,3 Hz; $J_{ortho34}$ = 7,2 Hz); 6,294 (s; 1H; NH); 3,274 (t; 4H; Octyl-1; $J_{CH2CH2}$ = 7,4 Hz); 1,605 (tt; 4H; Octyl-2; $J_{CH2CH2}$ = 7,4 Hz); 1,294 (m; 20H; Octyl-3-7; $J_{CH2CH2}$ = 6,3 Hz); 0,882 (t; 6H, Octyl-8; $J_{CH2CH3}$ = 6,3 Hz) ppm.

Ferner wurden 0,23 g Phenylharnstoff, das sind 17 Mol% der eingesetzten Menge und 0,41 g Dioctylamin, das sind 17 Mol% der eingesetzten Menge, zurückgewonnen.

**Beispiel 1a - 1d**

Phenylharnstoff und Dioctylamin wurden unter verschiedenen Bedingungen unter kräftigem Rühren in Xylol erhitzt. Nach gemessenen Reaktionszeiten wurden der Mischung Proben entnommen, das Lösungsmittel der jeweiligen Probe wurde bei 65 °C im Vakuum abgedampft und der Rückstand in $CDCl_3$ aufgenommen, worin Phenylharnstoff praktisch unlöslich, N-Phenyl-N',N'-dioctylharnstoff und Dioctylamin dagegen vollständig löslich sind. Der ausgefallene Phenylharnstoff wurde abfiltriert und vom Filtrat wurde ein $^1$H-NMR-Spektrum angefertigt. Durch Integration der Signale der $-N-CH_2$-Protonen des Dioctylamins (2,75 ppm) und des gebildeten N-Phenyl-N',N'-dioctylharnstoffs (3,28 ppm), wurde das jeweilige molare Verhältnis des Dioctylamins zum gebildeten N-Phenyl-N',N'-dioctylharnstoff bestimmt, aus dem das Maß für den Umsatz abgeleitet wurde.

In Beispiel 1a wurde der Umsatz (U) bei verschiedenen Anfangskonzentrationen der Reaktanten Phenylharnstoff (A) und Dioctylamin (B) im Lösungsmittel gemessen, wobei die Messungen jeweils nach 15 Minuten erfolgten. Dabei wurden die in Tabelle 1a beschriebenen Werte erhalten:

Tabelle 1a

| A (Mol/l) | B (Mol/l) | U nach 15 Minuten (%) |
|---|---|---|
| 0,1 | 0,1 | 41,5 |
| 0,2 | 0,2 | 44,6 |
| 1,0 | 1,0 | 56,0 |

In Beispiel 1b wurde U bei verschiedenen molaren Einsatzverhältnissen der Reaktanten A und B gemessen, wobei die Messungen jeweils nach 15 Minuten erfolgten. Die Ergebnisse sind in Tabelle 1b beschrieben:

Tabelle 1b

| A (Mol/l) | B (Mol/l) | A:B | U nach 15 Minuten (%) |
|---|---|---|---|
| 0,1 | 0,1 | 1 : 1 | 41,5 |
| 0,1 | 0,2 | 1 : 2 | 40,4 |
| 0,1 | 0,5 | 1 : 5 | 56,0 |
| 0,1 | 1,0 | 1 :10 | 73,3 |
| 0,2 | 0,2 | 1 : 1 | 44,6 |
| 0,2 | 0,4 | 1 : 2 | 55,3 |
| 0,7 | 0,5 | 14:10 | 44,2 |
| 0,7 | 1,0 | 7 :10 | 59,6 |

In Beispiel 1c wurde U bei gleichen Anfangskonzentrationen der Reaktionen A und B nach verschiedenen Reaktionszeiten (t) gemessen. Dabei wurden die in Tabelle 1c beschriebenen Werte gemessen.

Tabelle 1c

| A (Mol/l) | B (Mol/L) | t (Minuten) | U (%) |
|---|---|---|---|
| 0,1 | 0,1 | 5 | 12,9 |
| 0,1 | 0,1 | 10 | 28,1 |
| 0,1 | 0,1 | 15 | 41,5 |
| 0,1 | 0,1 | 20 | 49,1 |
| 0,1 | 0,1 | 25 | 58,9 |
| 0,1 | 0,1 | 30 | 68,6 |
| 0,1 | 0,1 | 40 | 75,6 |
| 0,1 | 0,1 | 60 | 83,3 |

In Beispiel 1d wurde U bei zwei verschiedener Reaktionstemperaturen (T) nach verschiedenen Reaktionszeiten (t) gemessen, wobei die Anfangskonzentration der Reaktanten im Lösungsmittel jeweils 0,2 Mol/l betrug. Dabei wurden die in Tabelle 1d beschriebenen Werte gemessen:

| t (Minten) | T (°C) | Umsatz (%) |
|---|---|---|
| 3 | 120<br>130 | 1,6<br>11,7 |
| 13 | 120<br>130 | 8,9<br>29,4 |
| 23 | 120<br>130 | 16,6<br>42,1 |
| 33 | 120<br>130 | 22,7<br>47,2 |
| 43 | 120<br>130 | 29,3<br>53,9 |

## Beispiel 2

wurde wie im Beispiel 1 beschrieben, aber unter Verwendung von 4-Methoxyphenylharnstoff anstatt Phenylharnstoff ausgeführt, wobei **N-(4-Methoxyphenyl)-N',N'-dioctylharnstoff** erhalten wurde. Der Umsatz nach 30 Minuten betrug etwa 60 %.
$^1$H-NMR (200 MHz; DMSO; TMS): 7,944 (s; 1H; NH); 7,305 und 6,780 (m; je 2H; Methoxyphenyl-2,3,5 und 6; $J_{ortho}$ = 0,9 Hz); 3,681 (s; 3H; Methoxy-$CH_3$); 3,223 (t; 4H; Octyl-1; $J_{CH2CH2}$ = 7,2 Hz); 1,462 (m; 4H; Octyl-2; $J_{CH2CH2}$ = 7,2 Hz); 1,236 (m; 20H, Octyl-3-7; $J_{CH2CH3}$ = 6,4 Hz); 0,838 (t; 6H; Octyl-8; $J_{CH2CH3}$ = 6,4 Hz) ppm.

## Beispiel 3

wurde wie Beispiel 1, aber unter Verwendung von Diethylamin anstatt Dioctylamin und von 4-Chlorphenylharnstoff anstatt Phenylharnstoff ausgeführt, wobei **N-(4-Chlorphenyl)-N',N'-diethylharnstoff** erhalten wurde.
$^1$H-NMR (200 MHz; $CDCl_3$; TMS): 7,336 und 7,198 (m; je 2H; Phenyl-2,3,5 und 6; $J_{ortho}$ = 9,0 Hz); 6,473 (s; 1H; NH); 3,344 (q; 4H; Ethyl-1; $J_{CH2CH3}$ = 7,0 Hz); 1,191 (t; 6H; Ethyl-2; $J_{CH2CH3}$ = 7,0 Hz) ppm.

## Beispiel 4

wurde wie Beispiel 1, aber unter Verwendung von 4-Chlorphenylharnstoff anstatt Phenylharnstoff ausgeführt, wobei **N-(4-Chlorphenyl)-N',N'-dioctylharnstoff** erhalten wurde. Der Umsatz nach 30 Minuten betrug etwa 20 %.
$^1$H-NMR (200 MHz; DMSO; TMS): 8,231 (s; 1H; NH); 7,477 und 7,235 (m; je 2H; Chlorphenyl-2,3,5 und 6; $J_{ortho}$ = 8,9 Hz); 3,244 (t; 4H; Octyl-1; $J_{CH2CH2}$ = 7,2 Hz); 1,459 (m; 4H; Octyl-2; $J_{CH2CH2}$ = 7,2 Hz); 1,226 (m; 20H; Octyl-3-7; $J_{CH2CH3}$ = 6,4 Hz); 0,830 (t; 6H, Octyl-8; $J_{CH2CH3}$ = 6,4 Hz) ppm.

## Beispiel 5

1,36 g Phenylharnstoff (10 mMol) und 0,73 g tert.Butylamin (10mMol) wurden in 50 ml Xylol unter kräftigem Rühren auf Rückfluß erhitzt. Das dabei gebildete Ammoniak wurde als Gas abgeführt. Nach 30 Minuten war ein Umsatz von etwa 50 % erreicht, ohne daß Nebenprodukte entstanden waren und die Reaktion wurde abgebrochen, das Lösungsmittel und das nicht umgesetzte tert.Butylamin im Vakuum abgedampft und der Rückstand in Chloroform aufgenommen. Dabei ging der gebildete N-Phenyl-N'-tert.Butylharnstoff in Lösung, während der nicht umgesetzte Phenylharnstoff ausfiel und abfiltriert wurde. Die Chloroformlösung wurde abgedampft und der Rückstand aus Ethanol umkristallisiert, um nicht abgedampften tert.Butylharnstoff zu entfernen.

Dabei wurden 0,90 g **N-Phenyl-N'-tert.Butylharnstoff**, das sind 47 % der Theorie, mit einer Reinheit von praktisch 100 % erhalten.

$^1$H-NMR (200 MHz; DMSO; TMS): 8,205 (s; 1H; Phenyl-NH); 7,343 (m; 2H; Phenyl-2 und 6; $J_{ortho23}$ = 8,0 Hz); $J_{meta}$ = 1,1 Hz); 7,185 (m; 2H; Phenyl-3 und 5; $J_{ortho23}$ = 8,0 Hz; $J_{ortho34}$ = 7,3 Hz); 6,847 (m; 1H; Phenyl-4; $J_{meta}$ = 1,1 Hz; $J_{ortho34}$ = 7,3 Hz); 5,965 (s; 1H; tert.Butyl-NH); 1,280 (s; 9H; tert.Butyl-$CH_3$) ppm.

Ferner wurden 0,38 g tert.Butylamin, das sind 52 Mol% der eingesetzten Menge und 0,72 g Phenylharnstoff, das sind 53 Mol% der eingesetzten Menge zurückgewonnen.

## Beispiel 6

wurde wie Beispiel 1, aber unter Verwendung von Octadecylamin anstatt Dioctylamin ausgeführt, wobei **N-Phenyl-N'-octadecylharnstoff** erhalten wurde.
$^1$H-NMR (200 MHz; Pyridin-d5; TMS): 9,259 (s; 1H; Phenyl-NH); 7,913 (m; 2H; Phenyl-2 und 6; $J_{ortho23}$ = 7,5 Hz; $J_{meta}$ = 1,1 Hz); 7,328 (m; 2H; Phenyl-3 und 5; $J_{ortho23}$ = 7,5 Hz; $J_{ortho34}$ = 7,3 Hz); 7,000 (m; 1H; Phenyl-4; $J_{meta}$ = 1,1 Hz; $J_{ortho34}$ = 7,3 Hz); 6,564 (t; 1H; Octydecyl-NH; $J_{CH2NH}$ = 6,0 Hz); 3,466 (dt; 2H, Octydecyl-1; $J_{CH2NH}$ = 6,0 Hz; $J_{CH2CH2}$ = 6,9 Hz); 1,557 (tt; 2H; Octydecyl-2; $J_{CH2CH2}$ = 6,9 Hz); 1,274 (m; 30H; Octadecyl-3-17; $J_{CH2CH3}$ = 6,1 Hz); 0,873 (t; 3H; Octadecyl-18; $J_{CH2CH3}$ = 6,1 Hz) ppm.

## Beispiel 7

wurde wie Beispiel 5, aber unter Verwendung von 4-Methoxyphenylharnstoff anstatt Phenylharnstoff und Ethylamin, das eingeleitet wurde, anstatt tert.Butylamin, ausgeführt, wobei **N-(4-Methoxyphenyl)-N'-ethylharnstoff** erhalten wurde.
$^1$H-NMR (200 MHz; $CDCl_3$; TMS): 7,646 (s; 1H; Phenyl-NH); 7,108 und 6,732 (m; je 2H; Phenyl-2,3,5 und 6; Jortho = 8,9 Hz); 5,807 (t; 1H; Ethyl-NH; $J_{CH2NH}$ = 5,1 Hz); 3,710 (s; 3H; Methoxy-CH3); 3,150 (dq; 2H; Ethyl-1; $J_{CH2NH}$ = 5,1 Hz; $J_{CH2CH3}$ = 7,2 Hz) 1,029 (t; 3H; Ethyl-2; $J_{CH2CH3}$ = 7,2 Hz) ppm.

## Beispiel 8

wurde wie Beispiel 5, aber unter Verwendung von (4-N,N-Dimethylamino)phenylharnstoff anstatt Phenylharnstoff und Propylamin anstatt tert.Butylamin ausgeführt, wobei **N-(4-(N,N-Dimethylamino)-phenyl)-N'-propylharnstoff** erhalten wurde.
$^1$H-NMR (200 MHz; $CDCl_3$; TMS): 7,251 (s; 1H; Phenyl-NH); 7,105 (m; 2H, Phenyl-2 und 6; $J_{ortho}$ = 8,9 Hz); 6,627 (m; 2H; Phenyl-3 und 5; $J_{orhto}$ = 8,9 Hz); 5,566 (t; 1H; Propyl-NH; $J_{CH2NH}$ = 5,6 Hz); 3,082 (dt; 2H; Propyl-1; $J_{CH2NH}$ = 5,6 Hz; $J_{CH2CH2}$ = 7,2 Hz); 2,864 (s; 6H; Dimethylamino-$CH_3$); 1,420 (tq; 2H; Propyl-2; $J_{CH2CH2}$ = $J_{CH2CH3}$ = 7,2 Hz); 0,836 (t; 3H; Propyl-3; $J_{CH2CH3}$ = 7,2 Hz) ppm.

## Beispiel 9

wurde wie Beispiel 8, aber unter Verwendung von iso-Propylamin anstatt Propylamin ausgeführt, wobei **N-(4-(N,N-Dimethylamino)-phenyl)-N'-isopropylharnstoff** erhalten wurde.
$^1$H-NMR (200 MHz; DMSO; TMS): 7,890 (s; 1H; Phenyl-NH); 7,170 (m; 2H; Phenyl-2 und 6; $J_{ortho}$ = 9,0 Hz); 6,632 (m; 2H, Phenyl-3 und 5; $J_{ortho}$ = 9,0 Hz); 5,780 (d; 1H; Isopropyl-NH; $J_{CHNH}$ = 7,6 Hz); 3,728 (dse; 1H; Isopropyl-CH; $J_{CHNH}$ = 7,6 Hz; $J_{CHCH3}$ = 6,5 Hz); 2,780 (s, 6H, Dimethylamino-$CH_3$); 1,065 (d; 6H; Isopropyl-$CH_3$; $J_{CHCH3}$ = 6,5 Hz) ppm.

## Beispiel 10

1,36 g Phenylharnstoff (10 mMol) und 2,41 g 2,6-Diisopropylanilin (14 Mol) wurden in 50 ml Xylol gelöst und auf Rückfluß erhitzt. Das dabei gebildete Ammoniak wurde als Gas abgeführt. Nach 30 Minuten war ein Umsatz von 13 % erreicht, ohne daß Nebenprodukte entstanden waren.
Die Reaktionsmischung wurde auf Raumtemperatur abgekühlt, wobei der nicht umgesetzte Phenylharnstoff ausfiel. Nach dem Abfiltrieren wurde das Lösungsmittel abgedampft und der Rückstand aus Chloroform umkristallisiert.

Dabei wurden 0,38 g **N-Phenyl-N'-((2,6-diisopropyl)phenyl)-harnstoff**, das entspricht 13 % der Theorie, mit praktisch 100%iger Reinheit erhalten.
$^1$H-NMR (200 MHz; DMSO; TMS): 8,757 (s; 1H; Phenyl-NH); 8,318 (s; 1H; DIPP-NH); 7,467 (m; 2H; Phenyl-2 und 6; $J_{phenyl-ortho23}$ = 7,6 Hz; $J_{Phenyl-meta}$ = 1,0 Hz); 7,243 (m; 2H; Phenyl-3 und 5; $J_{Phenyl-orhto34}$ = 7,3 Hz; $J_{Phenyl-ortho23}$ = 7,6 Hz); 6,914 (m; 1H; Phenyl-4; $J_{Phenyl-ortho34}$ = 7,3 Hz; $J_{Phenyl-meta}$ = 1,0 Hz); 7,280 (m; 1H; DIPP-4; $J_{DIPP-ortho34}$ = 6,8 Hz); 7,166 (m; 2H; DIPP-3 und 5; $J_{Dipp-ortho34}$ = 6,8 Hz); 3,209 (se; 2H; Isopropyl-CH; $J_{CHCH3}$ = 6,9 Hz); 1,165 (d; 12H; Isopropyl-$CH_3$; $J_{CHCH3}$ = 6,9 Hz) ppm.

Ferner wurden 1,15 g nicht umgesetzter Phenylharnstoff, das sind 85 % der Einsatzmenge und 2,00 g 2,6-Diisopropylanilin, das sind 83 % der Einsatzmenge zurückgewonnen.

## Beispiel 11

wurde wie Beispiel 10, aber unter Verwendung von (4-Methoxyphenyl)-harnstoff anstatt Phenylharnstoff und N,N-Dimethyl-1,4-phenylendiamin anstatt 2,6-Diisopropylanilin ausgeführt, wobei **N-(4-Methoxyphenyl)-N'-(4-(N,N-dimethylamino)phenyl)harnstoff** erhalten wurde.

$^1$H-NMR (200 MHz; DMSO, TMS): 8,323 und 8,205 (2s; je 1H, NH); 7,262 (m; 2H; DMAP-2 und 6; $J_{DMPA-ortho}$ = 9,0 Hz); 7,352 und 6,852 (2m; je 2H; Methoxyphenyl-H; $J_{MP-ortho}$ = 9,0 Hz); 6,688 (m; 2H; DMAP-3 und 5; $J_{DMPA-ortho}$ = 9,0 Hz); 3,706 (s; 3H, Methoxy-$CH_3$); 2,819 (s; 6H; Dimethylamino-$CH_3$) ppm.

## Beispiel 12

wurde wie Beispiel 10, aber unter Verwendung von (N,N-Dimethyl-1,4)-phenylharnstoff anstatt Phenylharnstoff und 4-Chlorphenylamin anstatt 2,6-Diisopropylamin ausgeführt, wobei **N-(4-(N,N-Dimethylamino)-phenyl)-N'-(4-Chlorphenyl)harnstoff** erhalten wurde.

$^1$H-NMR (200 MHz; DMSO; TMS): 8,667 (s; 1H; Chlorphenyl-NH); 8,323 (s; 1H; DMAP-NH); 7,479 und 7,255 (2m; je 2H; Chlorphenyl-2,3,5 und 6; $J_{CP-ortho}$ = 8,9 Hz); 7,297 (m; 2H; DMAP-2 und 6; $J_{DMAP-ortho}$ = 9,0 Hz); 6,679 (m; 2H; DMAP-3 und 5; $J_{DMAP-ortho}$ = 9,0 Hz); 2,809 (s; 6H; Dimethylamino-$CH_3$) ppm.

## Beispiel 13

1,36 g Phenylharnstoff (10 mMol) und 0,71 g Pyrrolidin (10 mMol) wurden in 50 ml Xylol gelöst und unter kräftigem Rühren auf Rückfluß erhitzt. Das dabei gebildete Ammoniak wurde als Gas abgeführt. Nach 30 Minuten waren noch keine Nebenprodukte entstanden ($^1$H-NMR und $^{13}$C-NMR Kontrolle) und ein Umsatz von 40 % erreicht.

Das Lösungsmittel und das Amin wurden daraufhin im Vakuum abgedampft und der Rückstand mit Chloroform gut durchgerührt. Dabei ging das gebildete N-Pyrrolidincarbonsäureanilid in Lösung, während der Phenylharnstoff ausfiel. Nach dem Abfiltrieren wurde das Lösungsmittel im Vakuum abgedampft .

Dabei wurden 1,1 g **N-Pyrrolidincarbonsäureanilid,** das sind 58 % der Theorie mit einer Reinheit von praktisch 100 % erhalten.

$^1$H-NMR (200 MHz; $CDCl_3$; TMS): 7,414 (m; 2H; Phenyl-2 und 6; $J_{ortho}$ = 8,1 Hz; $J_{meta}$ = 1,2 Hz); 7,223 (m; 2H; Phenyl-3 und 5; $J_{ortho23}$ = 8,1 Hz; $J_{ortho34}$ = 7,5 Hz); 6,969 (m; 1H; Phenyl-4; $J_{meta}$ = 1,2 Hz; $J_{ortho34}$ = 7,5 Hz); 6,543 (s; 1H; NH); 3,380 (t; 4H; Pyrrolidin-2 und 5; $J_{Pyr23}$ = 6,7 Hz); 1,860 (tt; 4H; Pyrrolidin-3 und 4; $J_{Pyr23}$ = 6,7 Hz) ppm.

Ferner wurden 0,30 g nicht umgesetztes Pyrrolidin, das sind 42 Mol% der eingesetzten Menge und 0,57 g nicht umgesetzter Phenylharnstoff, das sind 42 Mol% der eingesetzten Menge, zurückgewonnen.

## Beispiel 14

wurde wie Beispiel 13, aber unter Verwendung von Morpholin anstatt Pyrrolidin ausgeführt, wobei **N-Morpholincarbonsäure-4-anilid** erhalten wurde. Der Umsatz nach 30 Minuten betrug etwa 85 %.

$^1$H-NMR (200 MHz; $CDCl_3$; TMS): 7,342 - 7,194 (m; 4H; Phenyl-2,3,5 und 6; $J_{ortho34}$ = 8,2 Hz; $J_{ortho23}$ = 7,3 Hz); 7,072 (s, 1H, NH), 7,062-6,984 (m; 1H; Phenyl-4; $J_{ortho34}$ = 8,2 Hz); 3,585 (t; 2H, Morpholin-$CH_2$-O; $J_{CH2CH2}$ = 4,7 Hz); 3,366 (t; 2H, Morpholin-$CH_2$-N; $J_{CH2CH2}$ = 4,7 Hz) ppm.

## Beispiel 15

Einer Lösung von 2,4-Toluoldiharnstoff in Xylol wurden pro Mol 2,4-Toluoldiharnstoff 2 Mol Ethylamin zugesetzt und die Lösung unter kräftigem Rühren auf Rückfluß erhitzt. Das im Verlauf der Reaktion gebildete Ammoniak wurde als Gas abgeführt. Nach 30 Minuten waren noch keine Nebenprodukte entstanden ($^1$H-NMR und $^{13}$C-NMR Kontrolle). Das Lösungsmittel und das Amin wurden daraufhin im Vakuum abdestilliert.

Dabei wurde 2,4-Bis-(-diethylaminocarbamoyl)-toluol erhalten.

$^1$H-NMR (300 MHz; $CDCl_3$; TMS): 7,605 (d;1H; Phenyl-3; $J_{meta}$ = 2,2 Hz); 7,389 (dd; 1H; Phenyl-5; $J_{meta}$ = 2,2 Hz; $J_{ortho}$ = 8,2Hz); 7,032 (d; 1H; Phenyl-6; $J_{ortho}$ = 8,2 Hz; 6,371 und 6,187 (2s; je 1H; NH); 3,409 -

3,292 (2q; je 4H; Ethyl-1; $J_{CH2CH3}$ = 7,2 Hz); 2,175 (s;3H; Toluol-$CH_3$); 1,254 - 1,157 (2t; je 6H; Ethyl-2; $J_{CH2CH3}$ = 7,2 Hz) ppm.

Ferner wurden in der Reaktionsmischung Toluol-2,4-diharnstoff, N,N-Diethyl-toluylen-2,4-diharnstoff und Ethylamin, aber keinerlei Nebenprodukte festgestellt.

**Beispiel 16**

wurde wie Beispiel 15, jedoch im Autoklaven unter Autogendruck bei 140 °C durchgeführt. Dabei wurde in der Reaktionsmischung neben dem gebildeten Ammoniak 2,4-Bis-(diethylaminocarbamoyl)-toluol, N,N-Diethyl-toluylen-2,4-diharnstoff 2,4-Toluoldiharnstoff und Ethylamin, aber keinerlei Nebenprodukte nachgewiesen.

**Patentansprüche**

1. Verfahren zur Herstellung reiner N,N'-unsymmetrisch substituierter Phenylharnstoffe der Formel

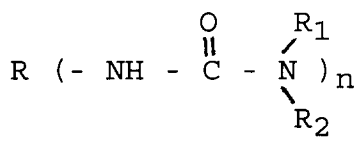

(I)

in der R eine unsubstituierte oder durch unter den Reaktionsbedingungen inerte Gruppen substituierte Phenyl- oder Phenylengruppe und $R_1$ und $R_2$ entweder gleich sind und jeweils eine Alkylgruppe, oder verschieden sind, wobei $R_1$ ein Wasserstoffatom oder eine Alkylgruppe und $R_2$ eine Alkyl- oder Phenylgruppe oder $R_1$ und $R_2$ gemeinsam mit dem Stickstoffatom einen heterocyclischen Ring, der keine ungesättigten Bindungen enthält, und n die Zahl 1 oder 2 bedeuten, durch Umsetzung eines Phenylharnstoffs der Formel

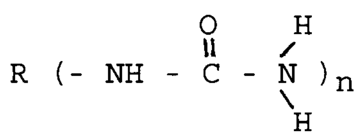

(II)

in der R und n die oben angegebende Bedeutung haben mit einem Amin der Formel

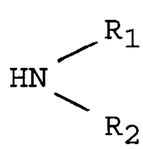

(III)

in der $R_1$ und $R_2$ die oben angegebenene Bedeutung haben, in einem unter den Reaktionsbedingungen inerten Verdünnungsmittel bei Temperaturen von 100 bis 200 °C dadurch gekennzeichnet, daß die Umsetzung, noch bevor Nebenprodukte entstehen, abgebrochen wird, worauf der N,N'-unsymmetrisch substituierte Phenylharnstoff der Formel I aus dem Reaktionsgemisch abgetrennt und die nicht umgesetzten Ausgangsverbindungen der Formeln II und III gegebenenfalls erneut in das Verfahren eingesetzt werden.

2. Verfahren nach Anspruch 1, dadurch gekennzeichnet, daß ein Harnstoff der Formel II eingesetzt wird, in der R einen unsubstituierten oder durch Halogenatome, Alkylgruppen mit 1 bis 6 C-Atomen oder Dialkylaminogruppen, wobei die Alkylgruppen 1 bis 3 C-Atome aufweisen, substituierte Phenylgruppe bedeuten.

3. Verfahren nach einem der Ansprüche 1 oder 2, dadurch gekennzeichnet, daß ein Amin der Formel III eingesetzt wird, in der $R_1$ und $R_2$ gleich sind und jeweils eine Alkylgruppe mit 1 bis 18 C-Atomen bedeutet.

4. Verfahren nach einem der Ansprüche 1 oder 2, dadurch gekennzeichnet, daß ein Amin der Formel III eingesetzt wird, in der $R_1$ ein Wasserstoffatom und $R_2$ eine Alkylgruppe mit 1 bis 22 C-Atomen oder eine unsubstituierte oder durch Halogen oder Dialkylaminogruppen, wobei die Alkygruppen 1 bis 3 C-Atome aufweisen, substituierte Phenylgruppe bedeutet.

5. Verfahren nach einem der Anspruch 1 oder 2, dadurch gekennzeichnet, daß ein Amin der allgemeinen Formel III eingesetzt wird, in der $R_1$ und $R_2$ gemeinsam mit dem Stickstoffatom einen heterocyclischen Ring, der keine ungesättigten Bindungen enthält, mit 5 oder 6 C-Atomen bedeuten, der durch Sauerstoff, Schwefel oder Stickstoff unterbrochen sein kann.

6. Verfahren nach einem der Ansprüche 1 bis 5, dadurch gekennzeichnet, daß der Harnstoff der Formel II und das Amin der Formel III in äquivalenten Mengen eingesetzt werden.

7. Verfahren nach einem der Ansprüche 1 bis 5, dadurch gekennzeichnet, daß die Reaktion bei Rückflußtemperatur des verwendeten inerten Verdünnungsmittels ausgeführt wird.

8. Verfahren nach einem der Ansprüche 1 bis 7, dadurch gekennzeichnet, daß das bei der Reaktion gebildete Ammoniak als Gas abgeführt wird.

9. Verfahren nach einem der Ansprüche 1 bis 8, dadurch gekennzeichnet, daß als inertes Verdünnungsmittel ein aromatischer Kohlenwasserstoff eingesetzt wird.

10. Verfahren nach einem der Ansprüche 1 bis 9, dadurch gekennzeichnet, daß der Zeitpunkt, ab dem Nebenprodukte auftreten, in einem Vorversuch ermittelt wird.

## Claims

1. Process for the preparation of pure N,N'-asymmetrically substituted phenylureas of the formula

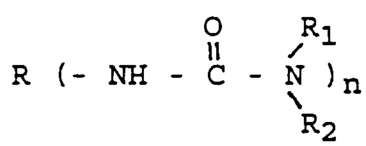

(I)

in which R represents a phenyl or phenylene group which is unsubstituted or substituted by groups which are inert under the reaction conditions and $R_1$ and $R_2$ are either identical and each represent an alkyl group or are different, $R_1$ representing a hydrogen atom or an alkyl group and $R_2$ representing an alkyl or phenyl group, or $R_1$ and $R_2$, together with the nitrogen atom, represent a heterocyclic ring which has no unsaturated bonds and n is the number 1 or 2, by reacting a phenylurea of the formula

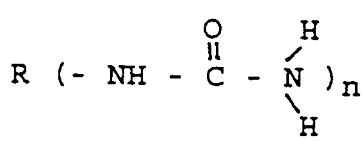

(II)

in which R and n have the abovementioned meaning, is reacted with an amine of the formula

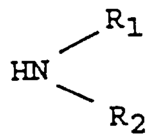

(III)

in which $R_1$ and $R_2$ have the abovementioned meaning, at temperatures from 100 to 200 °C in a diluent which is inert under the reaction conditions, characterized in that the reaction is terminated before by-products are formed, the N,N'-asymmetrically substituted phenylurea of the formula I is removed from the reaction mixture, and if appropriate the unreacted starting compounds of the formulae II and III are returned to the process.

2. Process according to Claim 1, characterized in that a urea of the formula II is used in which R represents a phenyl group which is unsubstituted or substituted by halogen atoms, alkyl groups with 1 to 6 C atoms or dialkylamino groups, the alkyl groups possessing 1 to 3 C atoms.

3. Process according to one of Claims 1 and 2, characterized in that an amine of the formula III is used in which $R_1$ and $R_2$ are identical and each represent an alkyl group with 1 to 18 C atoms.

4. Process according to one of Claims 1 and 2, characterized in that an amine of the formula III is used in which $R_1$ represents a hydrogen atom and $R_2$ represents an alkyl group with 1 to 22 C atoms or a phenyl group which is unsubstituted or substituted by halogen or dialkyamino groups, the alkyl groups possessing 1 to 3 C atoms.

5. Process according to one of Claims 1 and 2, characterized in that an amine of the general formula III is used in which $R_1$ and $R_2$, together with the nitrogen atom, represent a heterocyclic ring with 5 or 6 C atoms which has no unsaturated bonds and may be interrupted by oxygen, sulfur or nitrogen.

6. Process according to one of Claims 1 to 5, characterized in that the urea of the formula II and the amine of the formula III are used in equivalent amounts.

7. Process according to one of Claims 1 to 5, characterized in that the reaction is carried out at the reflux temperature of the inert diluent used.

8. Process according to one of Claims 1 to 7, characterized in that the ammonia formed during the reaction is removed as a gas.

9. Process according to one of Claims 1 to 8, characterized in that an aromatic hydrocarbon is used as inert diluent.

10. Process according to one of Claims 1 to 9, characterized in that the time from which by-products are produced is determined in a preliminary test.

**Revendications**

1. Procédé de préparation de phénylurées pures N,N'-suhstituées asymétriquement, répondant à la formule :

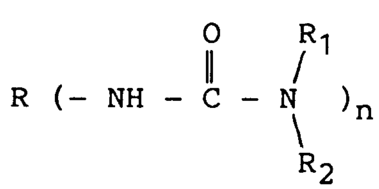

(I)

dans laquelle R est un groupe phényle ou phénylène non substitué ou substitué par des groupes

inertes dans les conditions de réaction, et $R_1$ et $R_2$ sont identiques et signifient chacun un groupe alkyle, ou ils sont différents l'un de l'autre et, dans ce cas, $R_1$ signifie un atome d'hydrogène ou un groupe alkyle, et $R_2$ signifie un groupe alkyle ou phényle, ou $R_1$ et $R_2$, conjointement avec l'atome d'azote, forment un noyau hétérocyclique qui ne contient pas de liaisons insaturées, et n vaut 1 ou 2, par mise en réaction d'une phénylurée de formule :

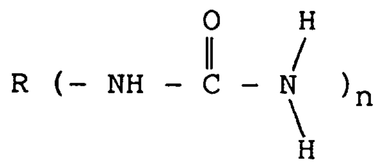

(II)

dans laquelle R et n ont la signification indiquée ci-dessus, avec une amine de formule :

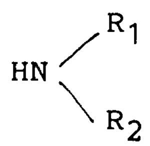

(III)

dans laquelle $R_1$ et $R_2$ ont la signification indiquée ci-dessus,
dans un diluant inerte dans les conditions de réaction, à des températures de 100 à 200 °C, caractérisé en ce qu'on interrompt la réaction avant la formation de produits secondaires, ensuite on sépare du mélange de réaction la phénylurée N,N'-substituée asymétriquement, de formule I, et, éventuellement, on recycle dans le procédé les composés de départ, de formule II et de formule III, qui n'ont pas subi de réaction.

**2.** Procédé selon la revendication 1, caractérisé en ce qu'on utilise une urée de formule II dans laquelle R signifie un groupe phényle non substitué ou substitué par des atomes d'halogène, des groupes alkyles ayant de 1 à 6 atomes de carbone ou des groupes dialkylamino dont les groupes alkyles contiennent de 1 à 3 atomes de carbone.

**3.** Procédé selon la revendication 1 ou 2, caractérisé en ce qu'on utilise une amine de formule III dans laquelle $R_1$ et $R_2$ sont identiques et signifient chacun un groupe alkyle ayant de 1 à 18 atomes de carbone.

**4.** Procédé selon la revendication 1 ou 2, caractérisé en ce qu'on utilise une amine de formule III dans laquelle $R_1$ signifie un atome d'hydrogène, et $R_2$ signifie un groupe alkyle ayant de 1 à 22 atomes de carbone ou un groupe phényle non substitué ou substitué par des atomes d'halogène ou par des groupes dialkylamino dont les groupes alkyles contiennent de 1 à 3 atomes de carbone.

**5.** Procédé selon la revendication 1 ou 2, caractérisé en ce qu'on utilise une amine de formule III dans laquelle $R_1$ et $R_2$ pris avec l'atome d'azote forment un noyau hétérocyclique qui ne contient pas de liaisons insaturées, contenant 5 ou 6 atomes de carbone, qui peut être interrompu par un atome d'oxygène, de soufre ou d'azote.

**6.** Procédé selon l'une quelconque des revendications 1 à 5, caractérisé en ce qu'on utilise des quantités équivalentes d'urée de formule II et d'amine de formule III.

**7.** Procédé selon l'une quelconque des revendications 1 à 5, caractérisé en ce qu'on effectue la réaction à la température de reflux du diluant inerte utilisé.

**8.** Procédé selon l'une quelconque des revendications 1 à 7, caractérisé en ce que l'ammoniac qui est formé pendant la réaction est évacué à l'état gazeux.

**9.** Procédé selon l'une quelconque des revendications 1 à 8, caractérisé en ce qu'on utilise un hydrocarbure aromatique comme diluant inerte.

**10.** Procédé selon l'une quelconque des revendications 1 à 9, caractérisé en ce qu'on détermine, lors d'un essai préalable, le moment à partir duquel des produits secondaires apparaissent.